# EUROPEAN PATENT APPLICATION

(11) **EP 4 289 372 A1**
(43) Date of publication of application: **13.12.2023**
(21) Application number: 22749162.8
(22) Date of filing: 29.01.2022
(51) Int. Cl.: A61B 17/22

(54) **ELECTRODE ASSEMBLY AND SHOCK WAVE APPARATUS USING SAME**

(30) Priority: 05.02.2021 CN 202110164455
(71) Applicant: Peijia Medical (Suzhou) Co., Ltd., Suzhou, Jiangsu 215000 (CN)
(72) Inventor: DING, Shangshang, Suzhou, Jiangsu 215000 (CN); CUI, Yuhu, Suzhou, Jiangsu 215000 (CN); TAN, Jian Fong, Suzhou, Jiangsu 215000 (CN); ZHANG, Yi, Suzhou, Jiangsu 215000 (CN)
(74) Representative: Engelhard, Markus
(86) International application number: PCT/CN2022/074971
(87) International publication number: WO 2022/166883

(57) **Abstract**

An electrode assembly for a shock wave apparatus. An electrode assembly (30) is disposed inside a balloon (10) of a shock wave apparatus (100). The electrode assembly (30) comprises: a first electrode (301); an insulating layer (302), the first electrode (301) being disposed inside the insulating layer (302) and the tail end of the first electrode (301) being exposed from the tail end of the insulating layer (302); a first electrical conductor (303), the first electrical conductor (303) being disposed on at least a portion of the outer peripheral surface of the tail end of the insulating layer (302); and a second electrode (304), the second electrode (304) being disposed on at least a portion of the outer peripheral surface of a base end of the insulating layer (302), such that an insulating gap is provided between the second electrode (304) and the first electrical conductor (303). Not only can the electrode assembly (30) effectively reduce the attenuation of a shock wave during a conduction process, but the service life of the electrode assembly (30) can also be effectively prolonged, thereby safely and reliably achieving a satisfactory therapeutic effect.

## Description

### RELATED APPLICATION DATA

The present application is the EP national phase of International Application No. PCT/CN2022/074971, filed on January 29, 2022 and claims the priority of Chinese application No. 2021101644557, filed on February 05, 2021.

### TECHNICAL FIELD

The present disclosure belongs to the technical field of medical techniques, in particular, to an electrode assembly used in a shock wave apparatus for treating cardiac valve or blood vessel calcification, a shock wave apparatus using the electrode assembly, and a method for treating cardiac valve or blood vessel calcification of an animal.

### BACKGROUND

Cardiac valve calcification is a main pathological manifestation of stenosis and regurgitation of a cardiac valve, etc., and usually taken place in elderly people. Blood vessel calcification is a generally common pathological manifestation of atherosclerosis, hypertension, diabetic vasculopathy, a vessel injury, a chronic renal disease and decrepitude, etc.

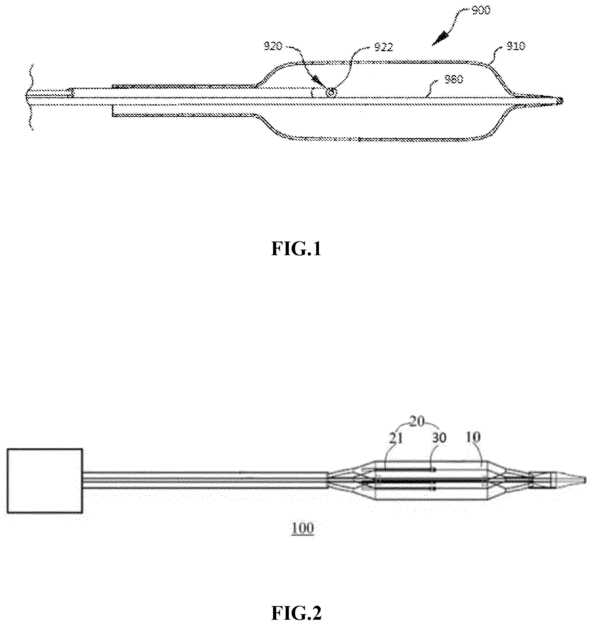

At present, a shock wave balloon technique has been used for treating cardiac valve or blood vessel calcification due to existence the advantages of being easily operated and performing pre-dilation by a balloon. As shown in FIG. 1, a shock wave apparatus 900 for treating cardiac valve calcification in the prior art includes a shock wave transmitter 920 and a balloon 910. The shock wave transmitter 920 comprises electrode cables which receive and conduct a voltage/current pulse and an electrode assembly 922 which is electrically connected to the electrode cables and is used for receiving the voltage/current pulse to generate shock wave. The balloon 910 is wrapped around the shock wave transmitter 920 from outside, and has stretchable, foldable and insulative properties. The balloon 910 is also provided with a through hole for the inflow of fluids, so that interior of the balloon 910 is filled with the fluid. The balloon 910 is dilated when the interior of the balloon 910 is filled with the fluid, so that at least a portion of outer surfaces of the balloon 910 comes into contact with a cardiac valve or blood vessel at which a calcification lesion is present (hereinafter sometimes abbreviated as "a calcification lesion portion" or "a cardiac valve or blood vessel having a calcification lesion"). The shock wave generated by the electrode assembly 922 is radially conducted to a surface of the balloon 910 through the liquid inside the balloon 910, and is then conducted to the calcification lesion portion through the surface of the balloon. When the shock wave is conducted to the calcification lesion portion, a compressive stress of the shock wave causes a fracture of calcified tissue at the calcification lesion portion. A shock wave of an appropriate intensity can achieve of breaking the calcified tissue without causing an additional burden of soft tissue around the calcified tissue.

However, the electrode assembly of the shock wave apparatus in the existing technique is mainly formed by a metal conductor and an insulating sheath; and higher energy is always required for the treatment of a cardiac valve calcification lesion, thus a voltage between a positive electrode and a negative electrode of a shock wave electrode may reach 7 KV to 10 KV, and the electrode generates intense thermal energy and mechanical energy during discharging, which causes a destructive impact to the structure of the shock wave electrode, bringing about great challenges to the service life of the shock wave electrode. In addition, in the case of severe cardiac valve or blood vessel calcification, it always leading to that it's difficult for the balloon of the shock wave apparatus to smoothly enter into the calcification lesion portion. Therefore, there are requirements for improvement in terms of the improving the treatment accuracy and effectiveness for the calcification lesion portion, the prolonging of the service life of the shock wave electrode in a high-voltage state, an optimized design of an electrode control system, etc.

In addition, a method of multi-channel control at low-voltage circuits is used in the shock wave apparatus that is provided with the plurality of balloons and/or the plurality of shock wave transmitters in the related art. In the control method, a multiple-channels control circuit is arranged ahead of a boost circuit, and each circuit needs a separate boost circuit and a separate high-voltage trigger switch. Since the volume of a boost component and the high-voltage trigger switch are relatively large, the overall circuit volume is relatively large, which results in a relatively large volume of the shock wave apparatus, thus causing the problem of inconvenient operation during an interventional surgery.

### SUMMARY

In view of the described technical problems in the related art, the present disclosure provides an electrode assembly which is used in a shock wave apparatus for treating cardiac valve or blood vessel calcification and by which the attenuation of a shock wave during conduction can be effectively reduced, and a satisfactory therapeutic effect is achieved for the cardiac valve or blood vessel calcification. In addition, by means of the electrode assembly of the present disclosure, the service life of an electrode and the shock wave apparatus can be significantly prolonged. The electrode assembly of the present disclosure can also enable the shock wave apparatus to quickly enter into a calcification lesion portion of a cardiac valve, thereby effectively shortening the time for a surgery. In addition, the present disclosure further provides a shock wave apparatus using the electrode assembly of the present disclosure, a control system for controlling the shock wave apparatus of the present disclosure, and a method for treating cardiac valve or blood vessel calcification using the shock wave apparatus of the present disclosure.

To solve the described technical problems, one aspect of embodiments of the present disclosure provides an electrode assembly for a shock wave apparatus which is disposed inside a balloon of the shock wave apparatus, and the electrode assembly includes:
a first electrode;
an insulating layer, the first electrode being disposed inside the insulating layer and a terminal end of the first electrode being exposed from a terminal end of the insulating layer;
a first electrical conductor disposed on at least a portion of an outer peripheral surface of the terminal end of the insulating layer; and
a second electrode disposed on at least a portion of an outer peripheral surface of a base end of the insulating layer, such that an insulating gap is provided between the second electrode and the first electrical conductor.

According to the electrode assembly of one aspect of the embodiments of the present disclosure, at least one of the first electrical conductor and the second electrode is provided with a protruding part which extends from one of the first electrical conductor and the second electrode to the other of the first electrical conductor and the second electrode along an outer peripheral surface of the insulating layer.

According to the electrode assembly in one aspect of the embodiments of the present disclosure, the electrode assembly further includes at least one second electrical conductor which is disposed on at least a portion of the outer peripheral surface of the insulating layer and is located between the first electrical conductor and the second electrode, such that an insulating gap is provided between the first electrical conductor and the second electrical conductor and between the second electrical conductor and the second electrode.

According to the electrode assembly in one aspect of the embodiments of the present disclosure, the at least one second electrical conductor is provided with a protruding part which extends from the second electrical conductor to the base end or the terminal end of the insulating layer along the outer peripheral surface of the insulating layer.

According to the electrode assembly in one aspect of the embodiments of the present disclosure, the first electrical conductor, the second electrical conductor and the second electrode are provided with two or more protruding parts in total, the two or more protruding parts are spaced apart from one another in a circumferential direction of the insulating layer by an angle of α, α = 360°/N, and N is the number of the protruding parts.

According to the electrode assembly in one aspect of the embodiments of the present disclosure, the first electrode can be moved inside the insulating layer in an axial direction, and
the terminal end of the first electrode is provided with a connecting part which makes the first electrode electrically connecting or electrically disconnecting the first electrical conductor during the movement of the first electrode.

According to the electrode assembly in one aspect of the embodiments of the present disclosure, the first electrode is a rod-shaped electrode having a diameter of 0.1 mm to 1.0 mm, preferably 0.1 mm to 0.5 mm.

According to the electrode assembly in one aspect of the embodiments of the present disclosure, the second electrode is an annular electrode having a wall thickness of 0.1 mm to 1.0 mm. According to the electrode assembly in one aspect of the embodiments of the present disclosure, each of the first electrical conductor and the second electrical conductor is an annular electrical conductor having a wall thickness of 0.1 mm to 1.0 mm.

According to the electrode assembly in one aspect of the embodiments of the present disclosure, the insulating layer is a cylindrical insulating sheath having a wall thickness of 0.1 mm to 1.0 mm.

Another aspect of the embodiments of the present disclosure provides a shock wave apparatus including the above-mentioned electrode assembly of the present disclosure.

Another aspect of the embodiments of the present disclosure provides a shock wave apparatus, the shock wave apparatus includes two or more balloons, and
at least one balloon of the two or more balloons is internally provided with the above-mentioned electrode assembly of the present disclosure.

Still another aspect of the embodiments of the present disclosure provides a method for treating cardiac valve calcification, the method uses the above-mentioned shock wave apparatus of the present disclosure to treat a calcified portion of a cardiac valve.

According to one embodiment of the present disclosure, an electrode assembly used in a shock wave apparatus for treating cardiac valve or blood vessel calcification can be provided. The electrode assembly can not only effectively reduce the attenuation of a shock wave during conduction, but also prolong the service life of the electrode assembly, thereby achieving a satisfactory therapeutic effect safely and reliably. In addition, the shock wave apparatus of the present disclosure is easily operated, and the requirement for the degree of operation proficiency of an operator is significantly reduced, such that the time for a surgery can be significantly shortened, and a burden of a treatment subject is alleviated, thereby improving the success rate of the surgery, and effectively avoiding various types of risks during the surgery.

### BRIEF DESCRIPTION OF THE DRAWINGS

In order to more clearly illustrate the technical solutions in the embodiments of the present disclosure, the drawings used in the description of the exemplary embodiments will be briefly described below. Obviously, the drawings in the following description are only some embodiments of the present disclosure, and an ordinary skilled technician in the art can obtain other drawings according to the drawings without involving any inventive effort.
FIG. 1 shows the structure of a shock wave apparatus in the prior art;
FIG. 2 shows the structure of one exemplary embodiment of a shock wave apparatus of the present disclosure;
FIG. 3 shows the usage state of one exemplary embodiment of the shock wave apparatus of the present disclosure;
FIG. 4 is a schematic cross-sectional diagram of a balloon portion of the shock wave apparatus of the present disclosure in an operation state;
FIG. 5 is a schematic diagram of an exemplary embodiment of an electrode assembly for a shock wave apparatus of the present disclosure;
FIGS. 6A and 6B are schematic diagrams of a modified embodiment of the electrode assembly for a shock wave apparatus of the present disclosure;
FIGS. 7A and 7B are schematic diagrams of a circuit control system in the modified exemplary embodiment of the electrode assembly for a shock wave apparatus of the present disclosure; and
FIG. 8 is a schematic diagram of a circuit control system of the shock wave apparatus of the present disclosure.

### DETAILED DESCRIPTION

The technical solutions in the embodiments of the present disclosure will be clearly and completely described below in conjunction with the drawings in the embodiments of the present disclosure. Apparently, the embodiments described are only some rather than all of the embodiments of the present disclosure. All other embodiments obtained by an ordinary skilled technician in the art based on the embodiments of the present disclosure without involving any inventive effort all fall within the scope of protection of the present disclosure.

In the present application, "shock wave" is a general term for various forms of waves (e.g. a pressure wave) that can be generated by an electrode assembly during charging, and is not used to define the specific form of wave.

In the present application, a "distal end" or a "terminal end" of a component of the shock wave apparatus or the electrode assembly or the like refers to an end that is a lead head end which enters into the body of a treatment subject during a surgery, and a "proximal end" or a "base end" of the shock wave apparatus or a component thereof is the end closer to a handle which is left outside of the body of the treatment body during the surgery compared to the "far end" and "end end".

In the present application, "a plurality of" refers to two or more. In view of this, "a plurality of" can be understood as "at least two" in the embodiments of the present disclosure. The term of "and/or" describes an association relationship between associated objects and represents that there may be three relationships. For example, A and/or B may represent: only A exists, both A and B exist, and only B exists. In addition, unless otherwise specified, the character of "/" generally indicates that the associated objects before and after the character are in an "or" relationship.

In the present application, "cardiac valve" and "valve" is a general term for valves including a mitral valve, a tricuspid valve and an aortic valve. In the present application, "a cardiac valve or blood vessel at which a calcification lesion is present" refers to "a cardiac valve and a blood vessel having a calcification lesion" or "a calcification lesion portion".

### Shock wave apparatus

As shown in FIG. 2, the shock wave apparatus 100 of one exemplary embodiment of the present disclosure may comprise at least one balloon 10. The balloon 10 is provided with at least one balloon body. Preferably, the balloon body of the balloon 10 is cylindrical after dilated. The shapes of two end parts of each balloon in a lengthwise direction are not specifically defined as long as the dilated balloon body of the balloon 10 is cylindrical. More preferably, the cylindrical balloon bodies of the balloon 10 are parallel to each other in the lengthwise direction. Specifically, axes of the cylindrical balloon bodies of the balloon 10 in the respective lengthwise directions are parallel to each other. The balloon 10 of the shock wave apparatus of the present disclosure may also have other shapes. For example, in one embodiment of the present disclosure, the balloon 10 may be provided with a plurality of balloon bodies. These balloon bodies may have the same shape, for example, a cylinder shape after being dilated with a liquid, and these balloon bodies are in communication with each other, such that the liquid can flow among these balloon bodies.

The balloon 10 may be formed from polymer materials as semi-compliance or non-compliance balloon with stretchable, foldable and insulative properties. The material forming the balloon 10 is not specifically limited, and may be, for example, nylon, polyether block amide (PEBA) or polyethylene terephthalate (PET), etc. The balloon 10 is also provided with at least one through hole which are in communication with a connecting tube A14 and is used for injecting liquid into the balloon 10 so that the balloon 10 is dilated. After the interior of the balloon 10 is filled with the fluid, the balloon is dilated, such that at least a portion of an outer surface of the balloon 10 comes into contact with a cardiac valve or blood vessel having a calcification lesion (a calcification lesion portion).

The balloon 10 is internally disposed with at least one shock wave transmitter 20 which is used for receiving a voltage/current pulse and generating shock wave. Preferably, as shown in FIG. 2, each balloon body of the balloon 10 is internally disposed with at least one shock wave transmitter 20. The shock wave transmitter 20 comprises at least one an electrode cable 21 which receives and conducts a voltage/current pulse and at least one electrode assembly 30 which is electrically connected to the electrode cable 21 and is used for receiving the voltage/current pulse to generate a shock wave. The shock wave generated by the electrode assembly 30 is radially conducted to a surface of the balloon 10 through the liquid inside the balloon 10, and is then conducted to a calcification lesion portion through the surface of the balloon.

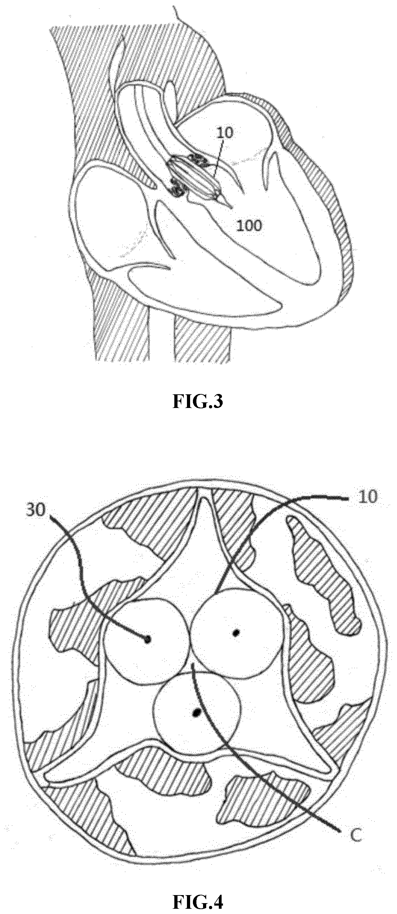

As shown in FIG. 3, during a surgery, the balloon 10 of the shock wave apparatus 100 is positioned at a cardiac valve position, for example. Preferably, the position of the electrode assembly 30 inside the balloon 10 corresponds to the calcification lesion portion, such that the distance between the electrode assembly 30 and the calcification lesion portion is the shortest. FIG. 4 shows a schematic cross-sectional diagram of a balloon portion of the shock wave apparatus 100 shown in FIG. 3 in operation state (dilated). As shown in FIG. 4, each balloon 10 includes a shock wave transmitter 20 which is disposed inside the balloon body of the balloon. Therefore, compared with the shock wave apparatus 900 in the prior art shown in FIG. 1, when the shock wave apparatus 100 of one exemplary embodiment of the present disclosure is in operation state, the distance between the electrode assembly 30 of the shock wave transmitter 20 that generates shock wave and a portion of the outer surface of the balloon 10 that is in contact with the calcification lesion portion is significantly shortened. Therefore, a shock wave generated by a lower voltage/current pulse can maintain sufficient intensity even though the shock wave is conducted to the calcification lesion portion, thereby obtaining a satisfactory therapeutic effect.

In another aspect, since the shock wave apparatus 100 of the present disclosure have a structure described above, that is, the cylindrical balloon bodies of each balloon 10 in the shock wave apparatus 100 are parallel to each other, the balloons are not prone to displacement caused by the slight pressing of a valve when the balloons are in contact with the calcification lesion portion during a surgery, and therefore, a healthcare worker can obtain a satisfactory therapeutic effect only by performing simple positioning operations. The requirement to operation proficiency level of the operator by the shock wave apparatus 100 in the embodiments of the present disclosure is significantly reduced, such that an operator having ordinary intervention surgery experience can proficiently operate the shock wave apparatus 100 of the present disclosure. Therefore, the time for a surgery can be significantly shortened, and a burden to a treatment subject is alleviated, thereby improving the success rate of the surgery, and reducing various types of risks during the surgery.

In another aspect, in one embodiment of the present disclosure, a plurality of balloons 10 are provided, and a gap for passage of blood is provided between or among balloons 10, such that a surgery can be performed in a case of ensuring the blood to flow without blocking, thereby alleviating a burden to a treatment subject caused by the surgery. Specifically, as shown in FIG. 4, after the plurality of balloons 10 (three in the figure) are dilated, an adequate gap is reserved in both a balloon outer-side region and a balloon inner-side region (portion C in FIG. 4). In an exemplary embodiment of the present disclosure as shown in FIG. 4, the shock wave apparatus 100 is provided with three balloons 10. However, in the other embodiments of the present disclosure, there may be two, four or more balloons 10.

In the shock wave apparatus 100 of an exemplary embodiment of the present disclosure, the diameter of a body portion of a balloon 10 is 2 mm to 12 mm. The diameter of the balloon 10 is preferably 6 mm to 10 mm, and more preferably 8 mm to 10 mm. When the diameter of the balloon is greater than 12 mm, the distance between the electrode assembly and the calcification lesion portion increases, posing a risk of over-attenuation of the intensity of shock wave. The length of the body portion of the balloon 10 is 20 mm to 60 mm, for example, 20 mm, 35 mm, 40 mm, 55 mm or 60 mm. When the balloon 10 is excessively long, there is a possibility of damage to heart tissue during the surgery, and it is too difficult for the shock wave apparatus to pass a bend when being delivered during the intervention surgery. On the other hand, when the balloon 10 is excessively short, it will be more difficult for operator to position the shock wave apparatus during the surgery.

The fluid which is used in the shock wave apparatus 100 and is injected into the balloon 10 is not specifically limited. The liquid may be an electrolyte liquid, for example, normal saline, etc., or the liquid may also be a non-electrolyte liquid, for example, glycerinum, etc.

The balloon 10 of the shock wave apparatus 100 of the present disclosure may be designed as a disposable consumable or a consumable that can be repeatedly used. When the balloon is the consumable that can be repeatedly used, disinfection and sterilization are required before using it. Moreover, the shock wave apparatus 100 of the present disclosure is provided with a plurality of separate balloons 10, and therefore, when one balloon is broken, it is possible to replace the broken balloon only, instead of discarding the entire shock wave apparatus, thereby significantly reducing the cost of a maintenance fee of the shock wave apparatus.

### Electrode assembly 30

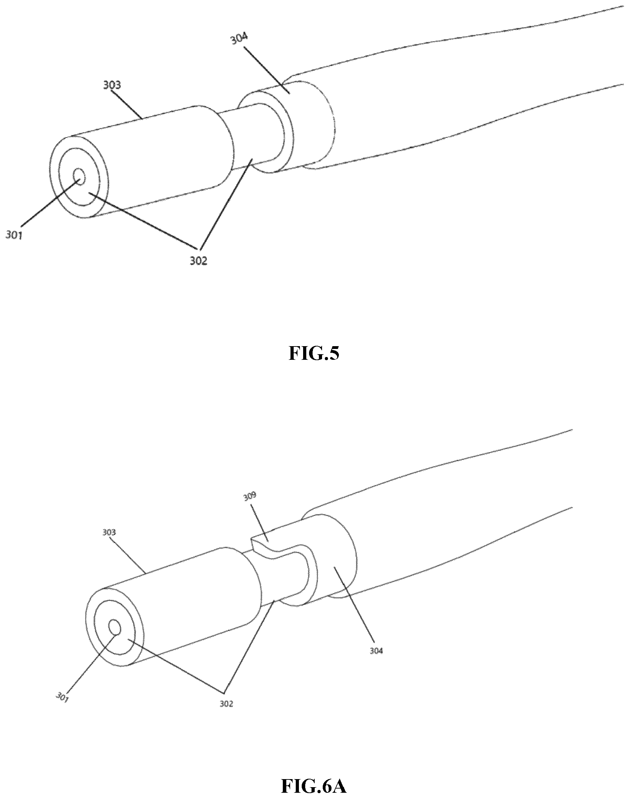

FIG. 5 shows an exemplary example of the electrode assembly of the present disclosure. As shown in FIG. 5, in one exemplary embodiment of the present disclosure, the electrode assembly 30 comprises an inner electrode 301, an insulating layer 302, a first electrical conductor 303 and an outer electrode 304.

The inner electrode 301 is a first electrode, which is disposed on the innermost layer of the electrode assembly 30, and is electrically connected to a power supply unit by means of a wire of the electrode cable 21. The material forming the inner electrode 301 is not specifically limited, may be any electrical conductor, but is preferably metal materials such as copper, silver, tungsten, etc. The shape of the inner electrode 201 is not specifically limited. However, as shown in FIG. 5, the inner electrode preferably has a rod shape with a circular cross section. The diameter of the rod-shaped inner electrode 301 is 0.1 mm to 1.0 mm, and preferably 0.1 mm to 0.5 mm.

The insulating layer 302 covers at least a portion of an outer peripheral surface of the inner electrode 301, such that a terminal end of the inner electrode 301 is exposed from a terminal end of the insulating layer 302. The terminal end of the inner electrode 301 may also be aligned with the terminal end of the insulating layer 302, alternatively, the terminal end of the inner electrode 301 may protrude from the terminal end of the outer insulating layer 302. The material forming the insulating layer 302 is not specifically limited and may be any insulator, but is preferably high-voltage resistant material such as polyimide or polytetrafluoroethylene. The shape of the insulating layer 302 is not specifically limited, as shown in FIG. 5, while is preferably an insulating sheath that covers the outer peripheral surface of the inner electrode 301 and has an annular cross section. The wall thickness of the annular insulating sheath is 0.1 mm to 1.0 mm, and preferably 0.2 mm to 0.5 mm. The length of the inner electrode 301 that is exposed from the distal end of the insulating layer 302 is not specifically defined.

The first electrical conductor 303 is disposed at the terminal end of the insulating layer 302 and covers at least a portion of the outer peripheral surface of the insulating layer 302. The first electrical conductor is not electrically connected to the power supply unit, and thus does not have any polarity. The material for forming the first electrical conductor 303 is not specifically limited, may be any conductor, but is preferably metal materials such as stainless steel, copper, etc. The shape of the first electrical conductor 303 is not specifically limited. However, as shown in FIG. 5, the first electrical conductor preferably has an annular shape that covers the outer peripheral surface of the terminal end of the insulating layer 302. The wall thickness of the annular first electrical conductor 303 is 0.1 mm to 1.0 mm.

Compared to the first electrical conductor 303, the outer electrode 304 is disposed at the base end of the insulating layer 302 spaced apart from the first electrical conductor 303 by a certain distance, , and covers at least a portion of the outer peripheral surface of the insulating layer 302. The outer electrode 304 is a second electrode, which is electrically connected to the power supply unit by means of one wire of the electrode cable 21. The material for forming the outer electrode 304 is not specifically limited, but is preferably metal materials such as stainless steel, copper, etc. The shape of the outer electrode 304 is not specifically limited, as shown in FIG. 5, while preferably has an annular shape that covers the outer peripheral surface of the base end of the insulating layer 302. The wall thickness of the annular outer electrode 304 is 0.1 mm to 1.0 mm. As shown in FIG. 5, an insulating gap is provided between the first electrical conductor 303 and the outer electrode 304 which are disposed on the outer peripheral surface of the insulating layer 302.

Based on the described electrode assembly 30, when a voltage is applied between the inner electrode 301 and the outer electrode 304, due to a voltage difference formed between the inner electrode 301 and the outer electrode 304 and according to voltage dividing principle in series circuits, voltage differences also exist in a gap (a first discharge point) between the terminal end of the inner electrode 301 and the first electrical conductor 303 and in a gap (a second discharge point) between the first electrical conductor 303 and the outer electrode 304. Mediums at the first discharge point and the second discharge point are broken down at the same time and shock wave energy is generated due to the presence of above voltage differences. Shock wave energy generated at the first discharge point (a head end of the electrode assembly 30) is radially conducted in an axial direction of the electrode assembly 30, while shock wave energy generated at the second discharge point is radially conducted in a radial direction of the electrode assembly 30.

Satisfactory effects can be obtained when the manner of discharge and shock wave conduction described above is used for treating severer cardiac valve and blood vessel calcification, especially severe cardiac valve calcification. When severe calcification occurs at, for example, a cardiac valve, it is difficult for the balloon of the shock wave apparatus to enter into a valvular annulus to dilate the valve. Therefore, when the shock wave apparatus which is provided with the electrode assembly 30 of the present disclosure is used, the shock wave which is generated at the first discharge point is conducted forward in the axial direction of the electrode assembly 30, so as to perform shock wave pre-dilation on a severely calcified valve, such that the balloon easily enters the valvular annulus. When the balloon of the shock wave apparatus completely enters into the valvular annulus, the shock wave which is generated at the second discharge point and is conducted in a radial direction of the electrode assembly 30 can further act on the calcification lesion portion. Therefore, more satisfactory effects can be obtained when the electrode assembly 30 of the present disclosure is used to treat a treatment subject having severe cardiac valve calcification, for example.

In an exemplary example of electrode assembly 30 shown in FIG. 5, a discharge gap (a discharge distance) D₁ at the first discharge point is constant, and D₁ is approximately the wall thickness of the insulating layer 302. A discharge gap D₂ at the second discharge point is not specifically limited. However, the sum of D₁ and D₂, i.e., the total discharge gap D (D = D₁ + D₂) should be smaller than the maximum distance Dₘₐₓ that can be broken down by an actual operation voltage. For example, taking pure water as a medium, Dₘₐₓ is approximately 1 mm when a voltage is 6 KV According to the electrode assembly 30 of the present disclosure, since D₁ is constant, the total discharge gap increases correspondingly as D₂ increases, such that the intensity of shock waves generated at the second discharge point can be enhanced. In one exemplary embodiment of the present disclosure, the length of D₂ is greater than the length of D₁, so that more energy are applied on the calcification lesion portion. In one exemplary embodiment of the present disclosure, D₂ can be appropriately adjusted in advance according to the degree of calcification of a cardiac valve or a blood vessel of a treatment subject. In another exemplary embodiment of the present disclosure, D₂ can be adjusted according to actual needs during a surgery to achieve an optimal therapeutic effect. For example, it is possible to make D₂ decrease to be smaller than D₁ during pre-dilation, so that energy is concentrated at the first discharge point; and after the balloon enters into a valvular annulus, it is possible to increase D₂ to be greater than D₁ (D₂ >D₁) , so that more energy is concentrated at the second discharge point. The specific method for adjusting D₂ is not specifically limited. For example, the outer electrode 304 may be connected to a control apparatus at a handle of the shock wave apparatus as shown in FIG. 2 by means of an insulating guide wire (not shown in the figures), such that the outer electrode 304 can be moved by means of the guide wire and the control apparatus, so as to adjust D₂. In one exemplary embodiment of the present disclosure, D₂ is 0.2 mm to 0.9 mm, and preferably 0.2 mm to 0.5 mm.

In one exemplary embodiment of the present disclosure, the outer electrode 304 and/or the first electrical conductor 303 may be provided with a protruding part 309. As shown in FIG. 6A, the protruding part 309 of the outer electrode 304 extends from the terminal end of the outer electrode 304 towards the first electrical conductor 303 along the outer surface of the insulating layer 302, but is not contact to the first electrical conductor 303. In another exemplary embodiment of the present disclosure, the first electrical conductor 303 may be provided with a protruding part 309 (not shown in the figures), which extends from the base end of the first electrical conductor 303 towards the outer electrode 304 along the outer surface of the insulating layer 302, but is not contact to the outer electrode 304. In still another embodiment of the present disclosure, each of the first electrical conductor 303 and the outer electrode 304 may be provided with a protruding part 309. By means of the provision of the protruding part 309, the discharge gap D₂ at the second discharge point becomes different in the entire circumferential direction of the electrode assembly 30. Specifically, D₂ becomes smaller at a position where the protruding part 309 is provided, and therefore, discharge also occurs between the protruding part 309 and the corresponding first electrical conductor 303/outer electrode 304, instead of randomly occurring across the entire second discharge point. According to the electrode assembly 30 of the described structure, the release and conduction directions of shock wave can be controlled, so as to better cooperate with the shock wave apparatus of the present disclosure that is provided with a plurality of balloons and to realize directed release of a shock wave, thereby further improving the quality and effect. The shape of the protruding part 309 is not specifically limited.

In one embodiment of the present disclosure, when the shock wave apparatus which is provided with the plurality of balloons is used, preferably, each balloon 10 is internally disposed with at least one electrode assembly 30. More preferably, the electrode assembly 30 in each balloon is provided with one protruding part 309. Still more preferably, the protruding part of each electrode assembly 30 is disposed at a side of the second discharge point that faces a position where the shock wave apparatus is in contact with the calcification lesion portion.

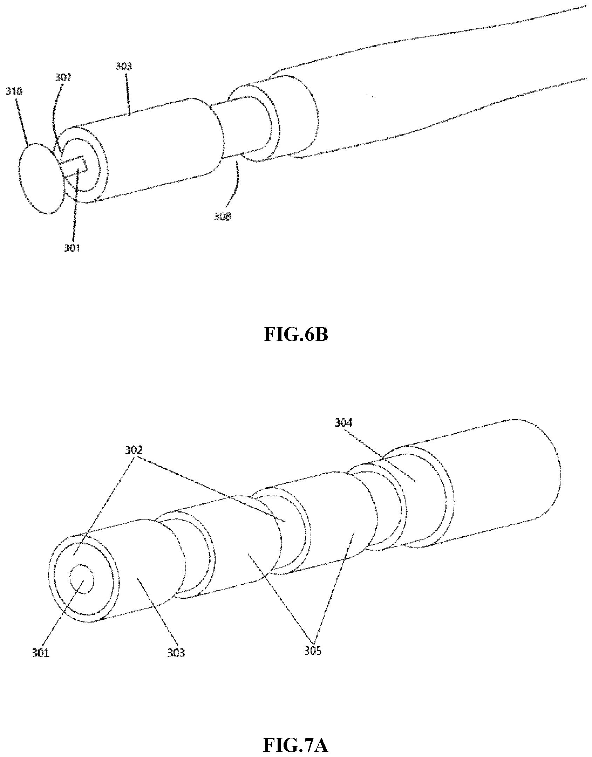

In one embodiment of the present disclosure, the inner electrode 301 can be moved inside the insulating layer 302 in the axial direction of the insulating layer 302. For example, the inner electrode 301 may be connected to the control apparatus at the handle (not shown in the figures) of the shock wave apparatus through an insulating guide wire (not shown in the figures), such that the inner electrode 301 can be moved by means of the guide wire and the control apparatus. In one embodiment of the present disclosure, the terminal end of the movable inner electrode 301 is provided with the connecting part 310, as shown in FIG. 6B. Preferably, the connecting part 310 can come into contact with the terminal end of the first electrical conductor 303 during the movement of the inner electrode 301, such that the inner electrode 301 is electrically connected to the first electrical conductor 303. According to the electrode assembly 30 of the described structure, when pre-dilation is completed and the balloon enters into the valvular annulus, the tip end (the first discharge point) of the electrode assembly 30 no longer needs to discharge. At this time, the discharge at the first discharge point is cancelled by means of the above-described electrical connection between the inner electrode 301 and the first electrical conductor 303, and discharge only occurs at the second discharge point. Therefore, the calcification lesion portion of the cardiac valve can be treated more effectively. The shape of the connecting part 310 is not specifically defined, as long as it realizes the above-described electrical connection between the inner electrode and the first electrical conductor 303.

In one embodiment of the present disclosure, in addition to the first electrical conductor 303, the electrode assembly 30 may further be provided with at least one second electrical conductor 305, as shown in FIG. 7A. The second electrical conductor 305 is disposed between the first electrical conductor 303 and the outer electrode 304. When more than two second electrical conductors 305 are provided, the second electrical conductors 305 are arranged at intervals between the first electrical conductor 303 and the outer electrode 304.

The first electrical conductor 303 and the second electrical conductors 305 (hereinafter sometimes collectively referred to as the electrical conductor) are not electrically connected to the power supply unit, and therefore do not have any polarity. The material for forming the second electrical conductor 305 is not specifically limited, may be any conductor, and preferably is metal materials such as stainless steel, copper, etc. The shape of the second electrical conductor 305 is not specifically limited, and the respective shapes of the first electrical conductor 303 and the second electrical conductors 305 may be the same or different. However, preferably, the second electrical conductors 305 have a shape of annular shape that covers a portion of the outer peripheral surface of the insulating layer 302, as shown in FIG. 7A. The wall thickness of each of the annular second electrical conductors is 0.1 mm to 1.0 mm. The number of second electrical conductors 305 in the electrode assembly 30 is not limited.

However, preferably, the electrode assembly 30 is provided with one to four second electrical conductors 305, and more preferably one to two of same.

As shown in FIG. 7A, an insulating gap is provided between the first electrical conductor 303 and the second electrical conductor 305 adjacent thereto, an insulating gap is provided between the outer electrode 304 and the second electrical conductor 305 adjacent thereto, and an insulating gap is provided between any two adjacent second electrical conductors 305.

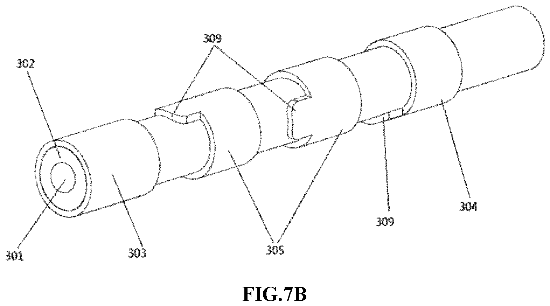

In one exemplary embodiment of the present disclosure, at least one second electrical conductor 30 may be provided with the protruding part 309, as shown in FIG. 7B. The arrangement of the protruding part 309 of the second electrical conductor 305 is similar to the protruding parts of the first electrical conductor 303 and the outer electrode 304, that is, the protruding part 309 extends from an end of the second electrical conductor 305 to the base end or the terminal end of the insulating layer 302 along the outer peripheral surface of the insulating layer 302, but is not in contact with the first electrical conductor 303/second electrical conductor 305/outer electrode 304 adjacent thereto.

According to the electrode assembly 30 of the present disclosure, when a treatment subject having severe cardiac valve and blood vessel calcification, and in particular severe cardiac valve calcification is treated, the balloon of the shock wave apparatus can easily enter into the valvular annulus by means of pre-dilation; moreover, after the balloon enters into the valvular annulus, the discharge of the head end of the electrode assembly 30 may be cancelled, such that energy is concentrated on the treatment of the calcification lesion portion.

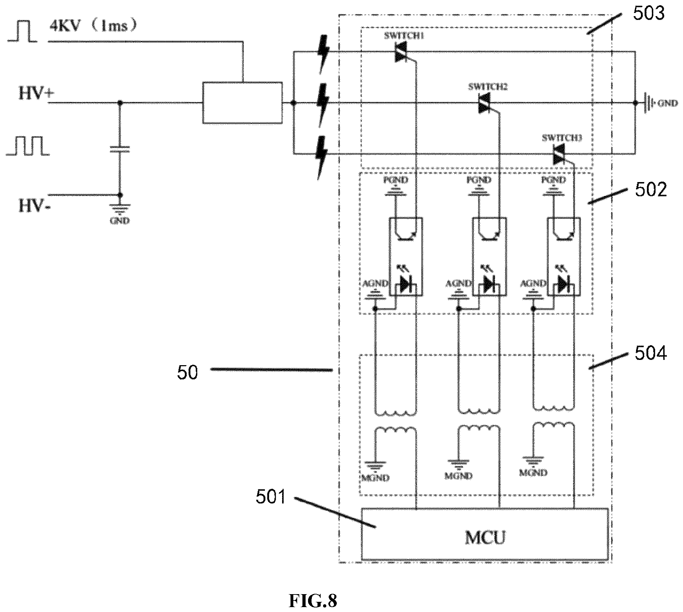

In one exemplary embodiment of the present disclosure, the shock wave apparatus 100 comprises a circuit control system 40 for controlling the shock wave transmitter 20. As shown in a dashed line box in FIG. 8, the circuit control system 40 of the shock wave apparatus of the present disclosure comprises a control unit 401, a high-voltage isolation unit 402 and a multi-channel control unit 403.

The control unit 401 is delayed by single chip microcomputer program software or controlled by a hardware timer circuit, and a plurality of output pins respectively correspond to multiple electrodes, so as to transmit a low-level signal, a high-level signal or a PWM signal, thereby realizing the control over a switch component in the multi-channel control unit 403.

The high-voltage isolation unit 402 is also referred to as a high-voltage isolation circuit. An electrode has an operation voltage of 1 KV to 20 KV when discharging, and thus generates a strong a peak voltage/current and electromagnetic interference during the discharge. However, the control unit 401 only has an operation voltage of 3 V to 5 V, and is poor in resistance to interference. In addition, a high-voltage switch device needs a larger driving voltage/current, and cannot be directly driven by a signal which is outputted by a single chip microcomputer, and thus it is necessary to boost a driving signal before performing control. The high-voltage isolation circuit 403 may use devices such as an optocoupler to enhance a driving capacity of an output signal of the single chip microcomputer of the control unit, while ensuring effective isolation between the control circuit and a load circuit, isolating a digital signal from an analog signal, and avoiding the high-voltage load circuit from interfering with the control circuit. The multi-channel control unit 403 is also referred to as a multi-channel separate control switch, which is used for controlling the switching on and off of a circuit. Specific examples of the multi-channel separate control switch include, but not limited to high-voltage switch devices such as a high-voltage relay, a high-voltage thyristor and a high-voltage IGBT. When the control unit 401 outputs a low-level signal, the switch is switched off; when the control unit 401 outputs a high-level signal, the switch is switched on; and when the control unit 401 outputs a PWM signal, the switch is periodically switched on and off. In the multi-channel control unit of the present disclosure, components need a larger parameter of withstand voltage and an overcurrent capacity greater than 20 A, and dv/dt should be greater than 7000 V/µs, so as to ensure the stability of switch performance. A high-voltage trigger switch is controlled by means of the multi-channel control unit 403, such that the discharge of the electrode assembly 30 is enabled by means of a high-voltage energy storage capacitor.

In one exemplary embodiment of the present disclosure, the circuit control system may also include a low-voltage isolation unit 404. The low-voltage isolation unit 404 is also referred to as a low-voltage isolation circuit, and specific examples thereof include a digital isolator, etc., which can satisfy an electrical safety standard or reduce noise of a grounding loop, etc. By means of the low-voltage isolation unit 404, dual-isolation between a digital signal and an analog signal can be realized, a carrying capacity of the control signal is improved, and it reduces or avoids influence of multiple optical isolation components on the speed and power consumption.

According to the circuit control system of the present disclosure in the exemplary embodiment, multi-channel control can be realized only by means of one boost circuit, and thus the overall volume of a high-voltage generator can be reduced by more than ten times, and a control circuit board can be realized only by means of a PCB with an area of 10 cm * 10 cm.

In one exemplary embodiment of the present disclosure, shock wave transmitters 20 in a plurality of balloons 10 may be respectively controlled during a surgery according to the actual condition of a treatment subject, so as to respectively generate shock waves of different intensities. The degrees of dilation of the plurality of balloons 10 may also be respectively controlled, such that the intensities of shock waves that are transmitted to calcification lesion portions having different degrees of calcification are respectively controlled. The above two methods may also be combined, generating/transmitting shock waves of different intensities for the calcification lesion portions having different degrees of calcification.

The structure and use of the shock wave apparatus of the present disclosure are described by taking human as a treatment subject in the exemplary embodiments of the present disclosure.

However, the treatment subject of the shock wave apparatus of the present disclosure is not limited to human, but may also be other animals. For example, an object of the patent for the shock wave apparatus of the present disclosure may be pets such as cats and dogs, may also be large animals such as cattle and horses, and may also be rare wild animals such as pandas. The foregoing are merely exemplary embodiments of the present disclosure, but are not intended to limit the patent scope of the present disclosure. Any transformation of equivalent structures or equivalent procedures made using the description and accompanying drawings of the present disclosure, which is directly or indirectly applied in other relevant technical fields, should similarly fall within the scope of patent protection of the present disclosure.

## Claims

1. An electrode assembly for a shock wave apparatus, the electrode assembly being disposed inside a balloon of the shock wave apparatus, and **characterized in** comprising:
a first electrode;
an insulating layer, the first electrode being disposed inside the insulating layer and a terminal end of the first electrode being exposed from a terminal end of the insulating layer;
a first electrical conductor disposed on at least a portion of an outer peripheral surface of the terminal end of the insulating layer; and
a second electrode disposed on at least a portion of an outer peripheral surface of a base end of the insulating layer, such that an insulating gap is provided between the second electrode and the first electrical conductor.

2. The electrode assembly according to claim 1, **characterized in that** at least one of the first electrical conductor and the second electrode is provided with a protruding part which extends from one of the first electrical conductor and the second electrode to the other of the first electrical conductor and the second electrode along an outer peripheral surface of the insulating layer.

3. The electrode assembly according to claim 1 or 2, **characterized in that** the electrode assembly further comprises at least one second electrical conductor which is disposed on at least a portion of the outer peripheral surface of the insulating layer and is located between the first electrical conductor and the second electrode, such that an insulating gap is provided between the first electrical conductor and the second electrical conductor and between the second electrical conductor and the second electrode.

4. The electrode assembly according to claim 3, **characterized in that** the at least one second electrical conductor is provided with a protruding part which extends from the second electrical conductor to the base end or the terminal end of the insulating layer along the outer peripheral surface of the insulating layer.

5. The electrode assembly according to claim 3 or 4, **characterized in that** the first electrical conductor, the second electrical conductor and the second electrode are provided with two or more protruding parts in total, the two or more protruding parts are spaced apart from one another in a circumferential direction of the insulating layer by an angle of α, α = 360°/N, and N is the number of the protruding parts.

6. The electrode assembly according to any one of claims 1 to 5, **characterized in that** the first electrode can be moved inside the insulating layer in an axial direction, and
the terminal end of the first electrode is provided with a connecting part which makes the first electrode electrically connecting or electrically disconnecting to the first electrical conductor during the movement of the first electrode.

7. The electrode assembly according to any one of claims 1 to 6, **characterized in that** the first electrode is a rod-shaped electrode having a diameter of 0.1 mm to 1.0 mm, preferably 0.1 mm to 0. 5 mm.

8. The electrode assembly according to any one of claims 1 to 7, **characterized in that** the second electrode is an annular electrode having a wall thickness of 0.1 mm to 1.0 mm.

9. The electrode assembly according to any one of claims 1 to 8, **characterized in that** each of the first electrical conductor and the second electrical conductor is an annular electrical conductor having a wall thickness of 0.1 mm to 1.0 mm.

10. The electrode assembly according to any one of claims 1 to 9, **characterized in that** the insulating layer is a cylindrical insulating sheath having a wall thickness of 0.1 mm to 1.0 mm.

11. A shock wave apparatus, **characterized in** comprising the electrode assembly according to any one of claims 1 to 10.

12. A shock wave apparatus, **characterized in that**
the shock wave apparatus comprises two or more balloons, and
at least one balloon of the two or more balloons is internally provided with the electrode assembly according to any one of claims 1 to 10.

13. A method for treating cardiac valve calcification, **characterized in** treating a calcified portion of a cardiac valve using the shock wave apparatus according to claim 11 or 12.
